(19) Europäisches Patentamt
European Patent Office
Office européen des brevets

(11) Veröffentlichungsnummer : **0 102 493 B1**

(12) # EUROPÄISCHE PATENTSCHRIFT

(45) Veröffentlichungstag der Patentschrift : **11.11.87**

(51) Int. Cl.⁴ : **B 01 J 23/78**, B 01 J 37/02, C 07 C 37/14, C 07 C 39/06

(21) Anmeldenummer : **83107086.7**

(22) Anmeldetag : **20.07.83**

(54) **Alkylierungskatalysator, Verfahren zur Herstellung dieses Katalysators sowie die Verwendung dieses Katalysators zur o-Alkylierung von Phenolen.**

(30) Priorität : **31.07.82 DE 3228713**

(43) Veröffentlichungstag der Anmeldung : **14.03.84 Patentblatt 84/11**

(45) Bekanntmachung des Hinweises auf die Patenterteilung : **11.11.87 Patentblatt 87/46**

(84) Benannte Vertragsstaaten : **DE FR GB NL**

(56) Entgegenhaltungen :
**EP-A- 0 019 476**
**DE-A- 2 428 056**
**FR-A- 812 290**
**US-A- 2 738 361**
**US-A- 2 778 805**
**US-A- 3 953 529**

(73) Patentinhaber : **BAYER AG**
**Konzernverwaltung RP Patentabteilung**
**D-5090 Leverkusen 1 Bayerwerk (DE)**

(72) Erfinder : **Käsbauer, Josef, Dr.**
**Morgengraben 1**
**D-5000 Köln 80 (DE)**
Erfinder : **Wedemeyer, Karlfried, Dr.**
**Bilharzstrasse 7**
**D-5000 Köln 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die vorliegende Erfindung betrifft einen Alkylierungskatalysator, ein Verfahren zu dessen Herstellung sowie die Verwendung des Katalysators zur o-Alkylierung von Phenolen.

o-Alkylierte Phenole sind wichtige organische Zwischenprodukte. So dient beispielsweise 2,6-Dimethylphenol als Zwischenprodukt zur Herstellung von methylsubstituierten Polycarbonaten und über seine Umwandlung in 2,6-Dimethylanilin zur Synthese von Pflanzenschutzmitteln.

Der wichtigste Syntheseweg zu solchen o-alkylierten Phenolen verläuft über die Gasphasenalkylierung von Phenol oder bereits einfach o-substituierten Phenolen mit niederen Alkoholen in Gegenwart eines Katalysators aus verschiedenen Metalloxiden. Die benötigten Temperaturen bewegen sich hierbei zwischen 350 und 600 °C. In den letzten Jahren erschien eine große Anzahl von Patentanmeldungen für diesen Syntheseweg, die vor allem die Zusammensetzung von verschiedenen Alkylierungs-Katalysatoren beschreiben. Die Ziele der beschriebenen Verfahren sind eine hohe Ausbeute und eine gute Selektivität bezüglich der Phenolalkylierung sowie möglichst kurze Aktivierungsphasen und lange Standzeiten der Katalysatoren.

Nach dem Verfahren von BE-A-888 904 sind Katalysatoren aus mindestens 4 Komponenten beschrieben, die Anspruch auf die o. g. Verbesserungen erheben, wobei neben Eisenoxid und Vanadinoxid eine Auswahl aus einer Vielzahl von metallischen und/oder nicht-metallischen Oxiden in einem Gemisch zur Anwendung kommen.

Als Nebenreaktion zur Alkylierung von Phenolen zersetzt sich das Alkylierungsreagens Alkanol zu Wasserstoff, Kohlendioxid, Kohlenmonoxid und zu dem dem Alkanol zugrundeliegenden Kohlenwasserstoff oder bei höheren Alkanolen dem zugrundeliegenden Olefin. Die Selektivität bezüglich des Alkanols wird in den meisten Patentanmeldungen nicht erwähnt und ist erfahrungsgemäß niedrig. Nach dem Verfahren von JP-A-56-45 427 (1981) gelingt unter technisch aufwendigen Bedingungen eine Reduzierung der Methanolzersetzung im günstigsten Fall von 46 % auf 38 %. Die Methanolzersetzung ist hierbei wie folgt definiert:

$$\frac{M_R - M_P - M_M}{M_R - M_M} \cdot 100 \ [\%]$$

wobei

$M_R$ das Methanol im Einsatzgemisch,

$M_P$ das Methanol im Produktgemisch und

$M_M$ das Methanol, das bei der Methylierung verbraucht wird,

darstellen, wobei alle Angaben in molaren Mengen gemacht werden.

Der JP-Anmeldung 56-49 330 (1981) ist zu entnehmen, daß die Methanolzersetzung von 97 % auf 66 % gesenkt werden kann. Als Reaktionstemperatur sind jedoch 420 °C nötig.

Überraschend wurde nun gefunden, daß ein Katalysator aus dem 3-Komponentensystem der Oxide des Eisens, des Siliciums und des Magnesiums neben hoher Ausbeute und Selektivität an o-substituierten und o,o'-disubstituierten Phenolen auch eine hohe Selektivität bezüglich des Alakanols zeigt. Durch die daraus resultierende geringere Abgasmenge und den geringeren Alkanolverbrauch liefert der neue Katalysator einen Beitrag zur Rohstoffersparnis und zum Umweltschutz. Außerdem wird kein Vanadium wie in BE-A-888 904 und kein Chrom wie in DE-A-24 28 056 benötigt, wodurch die Katalysatorkosten vorteilhaft niedrig gehalten werden können. Chromoxid wäre auch deshalb bedenklich, weil unter den Alkylierungsbedingungen die Bildung des cancerogenen Chromcarbonyls nicht ausgeschlossen werden kann. Beim erfindungsgemäßen Katalysator werden stark erniedrigte Alkanolzersetzungen beobachtet, so beispielsweise bei der Methylierung mit Methanol nur Methanolzersetzungen in der Nähe von 22 %.

Die Erfindung betrifft demnach einen Alkylierungskatalysator, enthaltend die Oxyde des Eisens, Siliciums und Magnesiums im Atomverhältnis Fe : Si : Mg = 100 : 0,1-10 : 0,1-15 sowie gegebenenfalls weiter enthaltend bis zu 5 % seines Gesamtgewichtes an Oxyden und/oder Carbonaten der Alkali- und/oder Erdalkalimetalle. In bevorzugter Ausführungsform enthält der Alkylierungskatalysator die Oxyde des Eisens, Siliciums und Magnesiums im Atomverhältnis Fe : Si : Mg = 100 : 0,2-6 : 0,1-10 sowie gegebenenfalls 0,1-5 % seines Gesamtgewichts an Oxyden und/oder Carbonaten der Alkali- und/oder Erdalkalimetalle.

Die Erfindung betrifft weiterhin ein Verfahren zur Herstellung des Alkylierungskatalysators. Zur Herstellung des Katalysators kann eine wäßrige Lösung eines Eisensalzes, eines Magnesiumsalzes und einer wasserlöslichen Siliciumberbindung, in der die genannten Elemente im Atomverhältnis Fe : Si : Mg = 100 : 0,1-10 : 0,1-15 enthalten sind, durch Zusatz einer alkalisch reagierenden Verbindung auf einen pH-Wert von 6 bis 9 eingestellt werden. Das sich bildende Gel läßt man sodann altern, wobei in bevorzugter Weise die Lösung gerührt wird. Das gealterte Gel wird sodann von der wäßrigen Phase, beispielsweise durch Filtration oder Zentrifugieren, getrennt, mit Wasser gewaschen, getrocknet und sodann calciniert.

Als wasserlösliche Einsensalze seien beispielsweise Fe-(III)-chlorid, Fe-(II)-nitrat, Fe-(II)-sulfat und Fe-(II)-chlorid, bevorzugt Fe-(III)-nitrat erwähnt.

Als wasserlösliche Siliciumverbindung seien beispielsweise Natriumsilikat und Kieselsäuresol erwähnt.

Als wasserlösliche Magnesiumsalze seien z. B. Magnesiumnitrat, Magnesiumchlorid, Magnesiumcarbonat und Magnesiumsulfat genannt.

Infolge der Hydrolyse beispielsweise der Fe-(III)-salze reagiert eine mit den geforderten Verhältnissen angesetzte Lösung der Fe-, Mg- und Si-Verbindungen vielfach schwach sauer. In einer solchen Lösung ist daher auch eine kleine Menge Mg-carbonat löslich, so daß auch dieses neben den oben erwähnten Mg-Salzen einsetzbar ist.

Die Salze werden im allgemeinen in dem Verhältnis in der wäßrigen Lösung eingesetzt, wie sie im Katalysator erwartet werden (angegebene Atomverhältnisse), da die gegenüber Eisen geringen Mengen an Mg- und Si-Verbindungen beim Einstellen des pH-Wertes vollständig mitgefällt werden. Nur bei größeren Mg- und/oder Si-Mengen, etwa im oberen Bereich der angegebenen Mengenverhältnisse kann es vorteilhaft sein, Mg- und/oder Si-Verbindungen in einem Überschuß beispielsweise bis zu 20 Gew.-% über der im Katalysator erwarteten Menge, einzusetzen. Auch ohne einen solchen Überschuß findet jedoch vollständige Ausfällung statt, wenn man den pH-Wert im oberen Teil des angegebenen Bereiches, etwa bei 8-9, einstellt.

Als alkalische Verbindung sei beispielsweise ein Hydroxid oder Carbonat eines Alkalimetalls, wie Lithium, Kalium, Natrium, oder das Hydroxid oder Carbonat eines Erdalkalimetalls, wie Calcium, mit Ausnahme des Magnesiums genannt. Diese alkalischen Verbindungen können beispielsweise als wäßrige Lösung oder Suspension zur Lösung des Einsensalzes, Magnesiumsalzes und der Siliciumverbindung gegeben werden.

Man läßt das durch die alkalische Verbindung ausgefällte Gel mit ausgefälltem Eisen, Magnesium und Silicium in Form beispielsweise der Oxide, Hydroxide, Oxidhydrate oder Carbonate zunächst altern. In bevorzugter Weise wird die mit der alkalischen Verbindung versetzte Lösung hierzu gerührt.

Das von der Lösung getrennte gealterte Gel wird sodann mit Wasser gewaschen, um den Hauptteil der alkalisch reagierenden Verbindung zu entfernen. Es ist jedoch nicht kritisch, wenn ein Teil dieser alkalisch reagierenden Verbindung absorptiv im Gel verbleibt und sodann nach dem Calcinieren einen Gehalt von bis zu 5 %, bevorzugt bis 2 % des Gesamtgewichts des Katalysators an Oxid, Hydroxid oder durch $CO_2$-Einwirkung gebildetes Carbonat des Alkali- oder Erdalkalimetalls ergibt. Frei von Alkali- oder Erdalkaliverbindung erhält man der erfindungsgemäßen Katalysator, wenn als alkalische Verbindung Ammoniaklösung verwendet wird.

Nach dem Trocknen bei 100-200 °C bei Normaldruck oder im Vakuum wird der Katalysator bei 350-700 °C calciniert. Der so erhaltene Katalysator kann durch Pressen, Granulieren, Zerkleinern und Sieben in eine für die o-Alkylierung gewünschte Form gebracht werden. Er hat eine lange Lebensdauer und kann bei Abnahme seiner Aktivität mit einem Sauerstoff enthaltenden Gas, beispielsweise Luft, durch Abbrennen regeneriert werden.

Die Erfindung betrifft außerdem die Verwendung des Alkylierungskatalysators zur o-Alkylierung von Phenolen durch Umsetzung von Phenolen mit mindestens einer freien o-Stellung mit niederen Alkoholen, gegebenenfalls in Gegenwart von Wasser, bei Temperaturen von 250-400 °C in der Gasphase.

Als erfindungsgemäß einsetzbare Alkanole seien solche mit 1 bis 4 Kohlenstoffatomen genannt, wie Methanol, Ethanol, Propanol, Isopropanol, Butanol oder Isobutanol. Bevorzugt werden Methanol und Ethanol, besonders bevorzugt Methanol, eingesetzt.

Als erfindungsgemäß einsetzbare Phenole mit mindestens einer freien o-Stellung sind solche der Formel

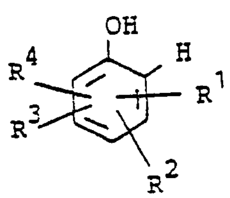

(I)

in der $R^1$ bis $R^4$ unabhängig voneinander Wasserstoff, $C_1$-$C_4$-Alkyl, Phenyl oder $C_4$-$C_7$-Cycloalkyl bedeuten und worin weiterhin zwei der genannten Reste gemeinsam die Trimethylen- oder Tetramethylengruppe bedeuten.

Beispiele für Verbindungen der Formel (I) sind Phenol, o-Kresol, m-Kresol, p-Kresol, 2,3-Xylenol, 2,4-Xylenol, 2,5-Xylenol, 3,5-Xylenol, 3,4-Xylenol, 2,3,4-Trimethylphenol, 2,3,5-Trimethylphenol, 2,4,5-Trimethylphenol, 2,3,4,5-Tetramethylphenol, o-Ethylphenol, m-Ethylphenol, p-Ethylphenol, 2,3-Diethylphenol, 2,4-Diethylphenol, 2,5-Diethylphenol, 3,5-Diethylphenol, 3,4-Diethylphenol, 2,3,4-Triethylphenol, 2,4,5-Triethylphenol, o-Propylphenol, o-Phenylphenol, p-Phenylphenol, o-Cyclohexylphenol, p-Cyclohexylphenol, 5-Hydroxy-1,2,3,4-Tetrahydronaphthalin, 4-Hydroxy-indan, 5-tert.-Butyl-3-methylphenol.

In bevorzugter Form werden erfindungsgemäß Phenole der Formel (II) eingesetzt

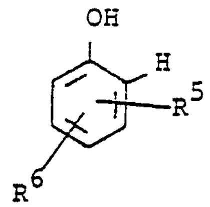

(II)

in der $R^5$ und $R^6$ unabhängig voneinander Wasserstoff, Methyl, Ethyl, Phenyl oder Cyclohexyl bedeuten.

Die Reaktion erfordert pro freie o-Stellung im Phenol 1 Mol Alkanol. Es kann jedoch auch ein Überschuß an Alkanol eingesetzt werden. Durch Zusatz von Wasser, beispielsweise 1 bis 3 Mol pro Mol Phenol, zur Reaktionslösung kann die Verkokung des Katalysators zurückgedrängt werden. Ebenso kann in die Reaktion nicht-alkyliertes Phenol oder nur teilweise o-alkyliertes Phenol zurückgeführt werden, das bei der Auftrennung des Reaktionsgemisches anfällt.

Die Temperatur im erfindungsgemäßen Verfahren liegt bei 250 bis 400 °C, bevorzugt bei 310 bis 380 °C. Der Reaktionsdruck liegt bei Atmosphärendruck, kann aber auch erhöht oder vermindert sein.

Beispiel 1

Zu einer Lösung von 980 g Eisennitrat $Fe(NO_3)_3 \cdot 9\ H_2O$, 3 g Magnesiumcarbonat (basisch) und 9,8 g Wasserglas in 9 l Wasser tropft man unter Rühren eine 10 %ige $NH_3$-Lösung bis sich ein pH-Wert von 7 einstellt. Nach einer Stunde Alterungszeit wird abgesaugt, dreimal mit Wasser gewaschen und bei 170 °C zehn Stunden im Luftstrom bei 66.6 kPa (500 Torr) getrocknet. Nach mechanischer Zerkleinerung werden die Korngrößen 0,5-1,0 mm und 1,0-2,0 mm herausgesiebt. Der Katalysator wird kurz bei 170 °C getrocknet und bei 470 °C sieben Stunden im Muffelofen kalziniert.

19 ml dieses Katalysators der Zusammensetzung Fe : Si : Mg = 100 : 1,6 : 1,3 (Atomverhältnis) werden in ein Glasrohr mit einem Außendurchmesser von 2 cm gefüllt. Korngröße 0,5-1,0 mm, Füllhöhe 12-13 cm. Das gefüllte Glasrohr wird in einen Ofen eingesetzt, der in mehreren Zonen heizbar ist, um über die gesamte Katalysatorstrecke isotherm zu fahren. Bei 330 °C wird über eine feine Kapillare eine Lösung von 1 Mol Phenol, 6 Mol Methanol und 1 Mol Wasser in das Reaktionsrohr gepumpt und auf einer Quarzbruchschicht, die über den Katalysator gefüllt ist, bei 250-300 °C verdampft und über den Katalysator geleitet. Das Reaktionsprodukt wird kondensiert und die Abgasmenge über eine Gasuhr registriert. Bei einem Durchsatz von LHSV (in Liter Einsatzlösung/1 Katalysator/Stunde) (liquid hourly space veolocity) = 1,1 $[h^{-1}]$ liegt die Ausbeute an 2,6-Dimethylphenol bei 96 % ; 22 % des Methanols werden zersetzt. Phenol wird vollständig umgesetzt.

Beispiel 2

Unter den gleichen Bedingungen wie in Beispiel 1 liefert eine Lösung von 1 Mol o-Kresol, 5 Mol Methanol und 1 Mol Wasser bei 325 °C, LHSV = 1,8 $[h^{-1}]$, 97 % 2,6-Dimethylphenol. 7 % des Methanols werden zersetzt.

Beispiel 3

Wie in Beispiel 1 setzt sich eine Lösung von 1 Mol m-Kresol, 6 Mol Methanol und 1 Mol Wasser bei 340 °C, LHSV = 0,9 $[h^{-1}]$, zu 93 % zu 2,3,6-Trimethylphenol um. 27 % des Methanols werden zersetzt.

Beispiel 4

Eine Lösung aus 1 Mol p-Kresol, 6 Mol Methanol und 1 Mol Wasser liefert unter den Bedingungen von Beispiel 1 bei 345 °C, LHSV = 1,2 $[h^{-1}]$, 94 % 2,4,6-Trimethylphenol. 30 % des Methanols werden zersetzt.

Beispiel 5

Eine Lösung von 1 Mol Phenol, 6 Mol Methanol und 1 Mol Wasser wird bei 250 °C verdampft und anschließend bei 330 °C über einen Katalysator, bestehend aus den Metalloxiden des Eisens, Siliciums und Magnesiums, Fe : Si : Mg = 100 : 2,5 : 0,1 (Atomverhältnis) geleitet. Bei LHSV = 1,0 $[h^{-1}]$ erhält man 94,5 % 2,6-Dimethylphenol bei 22 % Methanolzersetzung.

Beispiel 6

Das Einsatzgemisch wie in Beispiel 5 wird nach dem Verdampfen bei 250 °C bei einer Temperatur von 320 °C über einen Katalysator, bestehend aus den Oxiden des Eisens, Siliciums und Magnesiums, Fe : Si : Mg = 100 : 0,8 : 5,2 (Atomverhältnis) geleitet. Die Ausbeute an 2,6-Dimethylphenol liegt bei 95,5 % ; dabei zersetzen sich 32 % Methanol.

Beispiel 7

3,5-Dimethylphenol wird wie in Beispiel 1 mit Methanol bei 335 °C methyliert. Die Ausbeute an 2,3,5,6-Tetramethylphenol beträgt bei LHSV 1,1 [$h^{-1}$] 95 %.

Beispiel 8

Eine Lösung aus 1 Mol Phenol, 6 Mol Ethanol und 1 Mol Wasser wird bei 370 °C wie in Beispiel 1 umgesetzt. Bei LHSV = 0,9 [$h^{-1}$] ist die Selektivität der o-Ethylierung 84 % bei 87 % Umsatz.

Beispiel 9

1 Mol 5-tert.-Butyl-3-methylphenol wird mit 5 Mol Methanol in 1 Mol Wasser unter den Bedingungen des Beispiels 1 bei 350 °C mit einer Selektivität von 96 % in der o-Stellung zur phenolischen OH-Gruppe methyliert.

Beispiel 10 (Vergleich)

500 g $Fe(NO_3)_3 \cdot 9\ H_2O$ und 2,5 g $CaCO_3$ werden in 5 l Wasser gelöst. Anschließend tropft man eine Lösung aus 6,3 g Natriumsilikat in 100 ml Wasser zu. Die Salze werden durch langsames Zutropfen einer 10 %igen $NH_3$-Lösung ausgefällt (ca. pH = 8,0). Man rührt zur Alterung eine Stunde weiter, saugt das Gel ab und wäscht es dreimal mit je 5 l Wasser. Nach dem Trocknen bei 170 °C (40 kPa (300 Torr), 10 h) wird mechanisch zerkleinert und die Korngröße 0,5-1,0 mm abgesiebt. Man trocknet eventuell nochmals bei 170 °C (200 Torr, 2 h) und kalziniert 7 Stunden bei 450 °C. Der erhaltene Katalysator der Zusammensetzung Fe : Si : Ca = 100 : 2 : 2 (Atomverhältnis) wird wie in Beispiel 1 eingesetzt. Bei einer Katalysatorbett-Temperatur von 345 °C und bis zu einer Durchsatzleistung von LHSV = 1,76 $h^{-1}$ werden 93 % 2,6-Dimethylphenol erhalten. Die Methanolzersetzung liegt zwischen 1,8 und 2,7 Mol Methanol pro Mol gebildetes 2,6-Dimethylphenol.

Beispiel 11 (Vergleich)

Analog Beispiel 10 werden zur Katalysatorherstellung statt $CaCO_3$ 3,3 g $Ba(NO_3)_2$ verwendet. Der resultierende Katalysator der Zusammensetzung Fe : Si : Ba = 100 : 2 : 1 (Atomverhältnis) liefert unter den Bedingungen des Beispiels 1 bei 340 °C bis zu einer Durchsatzleistung von LHSV = 1,66 $h^{-1}$ 93-94 % 2,6-Dimethylphenol. 1,1 Mol Methanol wird pro Mol erhaltenes 2,6-Dimethylphenol zersetzt.

Beispiel 12

Ein weiterer Katalysator der Zusammensetzung Fe : Si : Mg : Ca = 100 : 1,3 : 2 : 2 wird aus 500 g $Fe(NO_3)_3 \cdot 9\ H_2O$, 4,1 g Wasserglas, 2,4 g Magnesiumcarbonat (basisch) und 2,5 g $CaCO_3$ analog Beispiel 10 hergestellt. Bei 335 °C werden bis LHSV = 1,25 $h^{-1}$ 94 % 2,6-Dimethylphenol erhalten. 0,8 Mol Methanol zersetzen sich pro Mol gebildetes 2,6-Dimethylphenol. Bei 340 °C liegt die Ausbeute bis LHSV = 1,23 $h^{-1}$ bei 96 %, wobei sich 1,9 Mol Methanol pro Mol 2,6-Dimethylphenol zersetzen.

**Patentansprüche**

1. Alkylierungskatalysator, enthaltend die Oxide des Eisens, Siliciums und Magnesiums im Atomverhältnis Fe : Si : Mg = 100 : 0,1-10 : 0,1-15 sowie gegebenenfalls weiter enthaltend bis zu 5 % seines Gesamtgewichts an Oxiden und/oder Carbonaten der Alkali- und/oder Erdalkalimetalle.

2. Alkylierungskatalysator nach Anspruch 1, dadurch gekennzeichnet, daß der Katalysator die genannten Oxide im Atomverhältnis Fe : Si : Mg = 100 : 0,2-6 : 0,1-10 sowie gegebenenfalls 0,1-3,0 % seines Gesamtgewichts an Oxiden und/oder Carbonaten der Alkali- und/oder Erdalkalimetalle enthält.

3. Verfahren zur Herstellung des Alkylierungskatalysators, der die Oxide des Eisens, Siliciums und Magnesiums im Atomverhältnis Fe : Si : Mg = 100 : 0,1-10 : 0,1-15 enthält sowie gegebenenfalls weiter bis zu 5 % seines Gesamtgewichts an Oxiden und/oder Carbonaten der Alkali- und/oder Erdalkalimetalle enthält, dadurch gekennzeichnet, daß man eine wäßrige Lösung eines Eisensalzes, eines Magnesiumsalzes und einer wasserlöslichen Siliciumverbindung, die die genannten Elemente im Atomverhältnis Fe : Si : Mg = 100 : 0,1-10 : 0,1-15 enthält, durch Zugabe von gegebenenfalls Hydroxyden und/oder Carbonaten der Alkali- und/oder Erdalkalimetalle auf einen pH-Wert von 6 bis 9 einstellt, das sich bildende

Gel altern läßt, dann von der wäßrigen Phase trennt, mit Wasser wascht, trocknet und calciniert.

4. Verwendung des Alkylierungskatalysators nach Anspruch 1 oder 2 zur o-Alkylierung von Phenolen, wobei Phenole mit mindestens einer freien o-Stellung mit niederen Alkanolen, gegebenenfalls in Gegenwart von Wasser, bei Temperaturen von 250 bis 400 °C in der Gasphase umgesetzt werden.

5. Verwendung des Alkylierungskatalysators nach Anspruch 1 oder 2 zur o-Methylierung von Phenolen.

**Claims**

1. Alkylation catalyst which contains the oxides of iron, silicon and magnesium in an atomic ratio of Fe : Si : Mg of 100 : 0.1-10 : 0.1-15 and, possibly, also contains up to 5 % of its total weight of oxides and/or carbonates of alkali and/or alkaline earth metals.

2. Alkylation catalyst according to Claim 1, characterised in that the catalyst contains the said oxides in an atomic ratio of Fe : Si : Mg of 100 : 0.2-6 : 0.1-10 and, possibly, 0.1-3.0 % of its total weight of oxides and/or carbonates of alkali and/or alkaline earth metals.

3. Process for preparing the alkylation catalyst which contains the oxides of iron, silicon and magnesium in an atomic ratio of Fe : Si : Mg of 100 : 0.1-10 : 0.1-15 and, possibly, also contains up to 5 % of its total weight of oxides and/or carbonates of alkali and/or alkaline earth metals, characterised in that an aqueous solution of an iron salt, a magnesium salt and a water-soluble silicon compound which contains the said elements in an atomic ratio of Fe : Si : Mg of 100 : 0.1-10 : 0.1-15, is brought to a pH value of 6 to 9 by adding, if appropriate, hydroxides and/or carbonates of alkali and or alkaline earth metals, and the gel which forms is allowed to age and is then separated from the aqueous phase, washed with water, dried and calcined.

4. Use of the alkylation catalyst according to Claim 1 or 2 for o-alkylating phenols, by reacting phenols which have at least one free o-position in the gas phase at temperatures of 250 to 400 °C with lower alkanols, if appropriate in the presence of water.

5. Use of the alkylation catalyst according to Claim 1 or 2 for o-methylating phenols.

**Revendications**

1. Catalyseur d'alkylation contenant les oxydes du fer, du silicium et du magnésium aux proportions atomiques Fe : Si : Mg = 100 : 0,1 à 10 : 0,1 à 15 et le cas échéant, en outre, jusqu'à 5 % de son poids total d'oxydes et/ou carbonates des métaux alcalins et/ou alcalino-terreux.

2. Catalyseur d'alkylation selon la revendication 1, caractérisé en ce qu'il contient les oxydes mentionnés aux proportions atomiques Fe : Si : Mg = 100 : 0,2 à 6 : 0,1 à 10 et le cas échéant 0,1 à 3,0 % de son poids total d'oxydes et/ou carbonates de métaux alcalins et/ou alcalino-terreux.

3. Procédé de préparation du catalyseur d'alkylation contenant les oxydes du fer, du silicium et du magnésium aux proportions atomiques Fe : Si : Mg = 100 : 0,1 à 10 : 0,1 à 15 et le cas échéant jusqu'à 5 % de son poids total d'oxydes et/ou carbonates des métaux alcalins et/ou alcalino-terreux, caractérisé en ce que l'on règle à un pH de 6 à 9 une solution aqueuse d'un sel de fer, d'un sel de magnésium et d'un composé hydrosoluble du silicium, contenant les éléments mentionnés aux proportions atomiques Fe : Si : Mg = 100 : 0,1 à 10 : 0,1 à 15, par addition le cas échéant d'hydroxydes et/ou de carbonates des métaux alcalins et/ou alcalinoterreux, on laisse vieillir le gel formé, on le sépare ensuite de la phase aqueuse, on le lave à l'eau, on le sèche et on le calcine.

4. Utilisation du catalyseur d'alkylation selon la revendication 1 ou 2 pour l'alkylation en ortho de phénols dans laquelle on fait réagir des phénols ayant au moins une position ortho libre avec des alcanols inférieurs, éventuellement en présence d'eau, à des températures de 250 à 400 °C, en phase gazeuse.

5. Utilisation du catalyseur d'alkylation selon la revendication 1 ou 2 pour la méthylation en ortho de phénols.